# EUROPÄISCHE PATENTSCHRIFT

(11) **EP 3 140 759 B1**
(45) Veröffentlichungstag und Bekanntmachung des Hinweises auf die Patenterteilung: **20.10.2021**
(21) Anmeldenummer: 15726510.9
(22) Anmeldetag: 24.04.2015
(51) Int. Cl.: A61B 5/00, A61H 31/00, A61M 16/00, G16H 40/63, G16H 50/20

(54) **PRÄKLINISCHES VERSORGUNGSSYSTEM**
PRECLINICAL CARE SYSTEM
SYSTÈME PRÉCLINIQUE DE PRESTATIONS DE SOIN

(30) Priorität: 07.05.2014 DE 102014006825
(43) Veröffentlichungstag der Anmeldung: 15.03.2017
(73) Patentinhaber: Weinmann Emergency Medical Technology GmbH + Co. KG, 22525 Hamburg (DE)
(72) Erfinder: HERRMANN, Frank, 25355 Barmstedt (DE); KALWA, Tobias, 20146 Hamburg (DE); KLÖCKNER, Felix, 22089 Hamburg (DE); PALM, Ulrich, 22848 Norderstedt (DE); SCOTTI, Norman, 20257 Hamburg (DE)
(74) Vertreter: Klickow & Wetzel PartGmbB
(86) Internationale Anmeldenummer: PCT/DE2015/000212
(87) Internationale Veröffentlichungsnummer: WO 2015/169274

(56) Entgegenhaltungen:
- EP-A1- 1 702 649
- US-A1- 2010 114 218
- US-A1- 2011 301 513
- US-A1- 2012 116 272
- None

## Beschreibung

Die Erfindung betrifft eine Vorrichtung zur präklinischen Notfallversorgung, die aus mindestens zwei miteinander vernetzten Modulen besteht, die durch die Vernetzung einen situationsabhängigen Handlungsablauf ermöglichen.

Die Erfindung betrifft insbesondere ein System bestehend aus präklinischen Notfallversorgungsmodulen, die durch Kombination in Verbindung mit einem menschlichen Notfallhelfer ein komplexes, möglichst ganzheitliches, zustandsabhängiges Regelungssystem ergeben.

Die Patentschrift US 2006111749 A1 beschreibt für den Basic Life Support ein System, dass einem Notfallhelfer sprachgeführt in einer kardio-pulmonalen Notsituation zur Seite steht. Hier stehen ein Defibrillator und ein Beatmungsgerät mit typischen Sensoren zur Verfügung, die mit Hilfe von Ablaufplänen die Notfallversorgung steuern.

In der Patentschrift US 6327497 B1 werden ein Verfahren und eine Vorrichtung zur Steuerung eines Defibrillators beschrieben, bei denen über einen Spannungsgenerator eine Elektroschockspannung an Elektroden angelegt wird. Zusätzlich wird von einer Druckgasflasche Sauerstoff zu einem Abgabeelement geleitet. Die Sauerstoffabgabe wird von einem Dosierelement zur Durchführung einer Oxygenierung gesteuert.

In der EP 1 369 144 B1 wird eine Vorrichtung zur Defibrillation, sowie eine Beatmungseinrichtung mit einer Steuerung für eine Zeitsynchronisation zur zeitlich aufeinander folgenden Durchführung von Inspirationsphasen und Exspirationsphasen einer Fibrillationsanalyse sowie einer anschließenden Defibrillation bei Erkennung eines Fibrillations-Zustandes beschrieben.

Die EP 1 264 615 B1 beschreibt ein Therapiegerät mit einer Beatmungseinheit, einer Defibrillationseinheit, einem Fibrillationsdetektor und einer Steuereinheit, die mit der Beatmungseinheit und der Steuereinheit verbunden und ausgebildet ist, zunächst die Beatmungseinheit zur Beatmung eines Patienten anzusteuern. Die beschriebene Einrichtung besteht auch in einem Therapiegerät, das ausgebildet ist, die Beatmung zur VF-Detektion durch den Fibrillationsdetektor zu unterbrechen und in Abhängigkeit der VF-Detektion die Defibrillationseinheit anzusteuern.

In der US 2012/011 62 72 A1 wird bereits die Kombination eines Beatmungsgerätes und eines Defibrillators beschrieben.

Aus der US 2010/011 42 18 A1 ist die Verwendung von vernetzten Komponenten im Bereich medizintechnischer Geräte bekannt.

Die US 2011/030 15 13 A1 beschreibt einen Geräteverbund, es ist jedoch kein Beatmungsgerät in diesem Verbund enthalten.

Aus der EP 17 02 649 A1 ist die Verwendung einer Basisstation bekannt, die im Zusammenhang mit einem medizintechnischen Gerät verwendet wird.

Allen bisher veröffentlichten Lösungsansätzen gemeinsam ist die Kombination von verschiedenen Modulen, die mit einander gekoppelt sind. Der Notfallhelfer wird oftmals durch akustische, optische oder textuelle Ausgaben in den Prozess mit eingebunden. Die Prozesse durchlaufen dabei einen oder mehrere festgelegte Anweisungspläne. Auf Modulebene hinterliegt eine Entscheidungslogik. Systemtechnisch ist diese aber nicht vorhanden.

Aufgabe der vorliegenden Erfindung ist es, eine Vorrichtung der einleitend genannten Art derart zu realisieren, dass auf Systemebene ein intelligentes Entscheidungssystem vorliegt, das mit und ohne den Notfallhelfer zu situationsoptimierten Maßnahmen führt.

Diese Aufgabe wird erfindungsgemäß durch die Merkmale von Patentanspruch 1 gelöst.

Diese Aufgabe wird insbesondere auch erfindungsgemäß dadurch gelöst, dass die Verbindbarkeit über Datenübertragungsmittel zum Austausch von Adress-, Zeit- und Statussignalen dient, wobei die Systemvorrichtung zumindest eine Sensoreinrichtung zur Gewinnung von Statussignalen und eine Zeitmesseinrichtung sowie einen Datenspeicher aufweist, sowie dass die Module derart ausgebildet sind, um ein Statussignal der jeweiligen Sensoreinrichtung mit einem Zeitsignal der Zeitmesseinrichtung zu verknüpfen und dass zumindest ein Modul der Systemvorrichtung diese Daten zusammen mit einer Adresskennung über zumindest eine Schnittstelle mittels eines Datenübertragungsprotokolls überträgt.

Erfindungsgemäß werden Zuordnungsdaten durch Wirkung der Steuermittel als Reaktion auf eine manuelle Betätigung, eine spezifische elektronische Ansteuerung, eine festgestellte Übertragungsstörung der Datenübertragungsmittel und/oder eine Aktivierung oder Deaktivierung eines Moduls automatisch und dynamisch generiert, geändert und/oder ergänzt sowie in elektronischen Speichermitteln abgelegt.

Dies soll insbesondere so durchgeführt werden, dass situations- und interaktionsabhängig die Module ein unterschiedliches Verhalten zeigen. Der Notfallhelfer kann dabei eingebunden werden. Abhängig von allen gewonnen Sensordaten, die unterschiedlich gewichtet werden, werden dem Notfallhelfer Entscheidungshilfen vorgeschlagen bzw. das System entscheidet selbsttätig. Um dies zu ermöglichen, steht die abgesicherte Kommunikation der Module im Vordergrund. Mit dem Notfallhelfer wird hierbei akustisch (Alarme und/oder Ansagen), optisch, haptisch oder über jede andere Art der menschlichen Wahrnehmung kommuniziert. Aktionen oder Feedback des Notfallhelfers nimmt das System ebenso über akustische Signale (Spracherkennung), Gesten oder Eingabesensoren entgegen.

Ein Modul ist zum Erzeugen, Senden und/oder Empfangen eines Bestätigungssignals für eine von einem anderen Modul durchgeführte Aktion ausgebildet.

Erfindungsgemäß ist eine erste Funktionseinheit mit ersten digitalen Adressdaten über die Datenübertragungsmittel ansteuerbar und identifizierbar. Eine jeweilige Aktoreinheit der ersten Funktionseinheit kann so mittels gespeicherter primärer Betriebsdaten angesteuert werden, wobei die primären Betriebsdaten im Bereich der ersten Funktionseinheit abrufbar gespeichert sind, oder durch, über die Datenübertragungsmittel empfangene und/oder empfangbare, sekundäre Betriebsdaten angesteuert werden. Sekundäre Betriebsdaten sind dann immer höher priorisiert.

Erfindungsgemäß ist mindestens eine der Funktionseinheiten so ausgebildet, dass sie modulartig mit spezifisch für leitungsgebundene und/oder drahtlose Datenübertragung über die Datenübertragungsmittel ausgebildeten Modulen aus, auf- und nachrüstbar ist.

Erfindungsgemäß weist die Systemvorrichtung eine leitungsgebundene Kommunikationsvorrichtung zur Verbindung von mindestens zwei Funktionseinheiten, und/oder eine drahtlose Kommunikationsvorrichtung zur Verbindung von mindestens zwei Funktionseinheiten auf.

Für die Verbindung der Module erweist sich ein Master/Slave-Konzept als günstig. Ein Master hat hiermit die Kontrolle über mindestens einen Slave. Der Slave agiert selbstständig auf seiner Modulebene (misst und/oder regelt in seinem Wirkbereich) und gibt seine Werte an den Master weiter. Der Master seinerseits kann über andere im System gesammelten Daten Einfluss auf den Slave nehmen, so wie die Slavedaten den Master beeinflussen können.

Ein Master kann als Slave einem höher priorisierten Master untergeordnet sein, so dass sich eine Hierarchiestruktur ergibt. Der Notfallhelfer kann dabei sowohl Master, als auch Slave sein. In der Regel wird es einen höchstpriorisierten Systemmaster geben, der durch ein beliebiges technisches Modul gebildet wird.

Während der Durchführung von Versorgungsmaßnahmen ist ein Wechsel des Systemmasters denkbar und in manchen Fällen sinnvoll. So könnte das technische System als Master agieren und den Helfer entlasten. Bei einer Änderung des Patientenzustands übernimmt aber der Helfer wieder eine Funktion. Oder im Fall einer Reanimation ist zunächst der Defibrillator der Master und übergibt bei erfolgreicher Herztätigkeit die Masterfunktion an das Beatmungsgerät, das CO2 und SpO2 abhängig die Beatmung steuert und die EKG Informationen zur Kontrolle erhält.

Für die sichere Kommunikation der Module untereinander ist eine Datenübertragung mit Checksummen o.ä. und Feedback notwendig. Ebenso notwendig ist die eindeutige Identifikation der Daten und die damit verbundene Zuordnung zu den Sendemodulen. Wichtig ist für alle Kommunikationsdaten der Erfassungszeitpunkt. Für den Fall, dass die Kommunikation gestört ist, wird immer wieder versucht, die Verbindung aufzubauen, es sei denn, die Verbindung wird aktiv durch den Nutzer abgebrochen. In der Zwischenzeit arbeiten die Module autark bzw. gehen in einen passiven Modus.

Besondere Bedeutung fällt der Kommunikation zum Notfallhelfer zu. Maßnahmen für die eindeutige Identifikation des Nutzers (Fingerabdrucksensor, RFID-Armband, Stimmenerkennung, Iris-Scan, etc.) sind hier hilfreich oder rechtlich sogar notwendig. Der akustischen Kommunikation vom und zum Helfer fällt hier eine große Bedeutung zu, damit der Notfallhelfer die Hände für seine Hilfsmaßnahmen frei hat. Auch Maßnahmen durch den Notfallhelfer sollten quittiert werden.

Eine spezielle Lösung könnte eine Datenbrille sein. Dem Notfallhelfer werden alle hilfreichen Informationen eingeblendet. Videodaten wie die eines Videolaryngoskops können eingeblendet werden. Durch Iris-Scan ist eine Identifikation möglich. Augenbewegungen und akustische Signale können der Kommunikation dienen.

Eine besondere Bedeutung hat die Handhabung von Alarmsignalen zu. Alarme, die auf Modulebene sinnvoll sind, können auf Systemebene eine völlig andere Priorität und Bedeutung haben. Der Systemmaster könnte hier die Alarmausgabe auf Modulebene beeinflussen und/oder priorisieren. Im Falle einer Reanimation würden z.B. während der Herzmassage, bestimmte Alarme des Beatmungsgeräts unterdrückt werden, bis es wieder zu gesteuerten Atemhüben kommt.

Eine Reanimationsvorrichtung besteht beispielhaft im Wesentlichen aus den Modulen 'Beatmungsgerät' und 'Defibrillator'. Die Kommunikation der Module erfolgt über ein Kabel oder per Funk und dient dem Austausch von Adress-, Zeit- und Statussignalen sowie Sensorsignalen. Das Beatmungsgerät weist als Slave-Elemente Druck- und Flusssensoren auf, die Druck- bzw. Flusssignale des Atemgases registrieren und an den Beatmungsmaster melden. Der Beatmungsmaster steuert mit diesen Informationen Aktoren, die als Slave arbeiten.

Der Defibrillator weist zwei Elektroden zur Gewinnung von Statussignalen der Herztätigkeit (EKG) auf. Das EKG-Modul arbeitet als Slave für die Steuereinheit (Master) des Defibrillators. Defibrillator und Beatmungsgerät verfügen zudem jeweils über eine Zeitmesseinrichtung. Diese dient dazu, eine Systemzeit zu generieren, welche - in Form eines Zeitstempels - mit den Sensorsignalen (Druck, Fluss, EKG) verknüpft wird.

Zudem weisen Defibrillator und Beatmungsgerät jeweils eine individuelle Adresskennung auf, über die die Geräte identifizierbar sind. Defibrillator und Beatmungsgerät weisen zudem jeweils eine Steuereinheit auf und sind ausgebildet, um über ein Datenübertragungsprotokoll die jeweiligen Sensordaten oder Steuersignale zum jeweils anderen Gerät zu übertragen.

Die Daten der Sensoren werden typisch mit der jeweiligen Adresskennung und dem jeweiligen Zeitstempel der jeweiligen Zeitmesseinrichtung verknüpft und dann entweder gespeichert und/oder über das Datenübertragungsmittel zum jeweils anderen Gerät übertragen. Oftmals werden diese Daten zusammen mit Steuersignalen übertragen, da die Sensordaten fallweise Ereignisse darstellen, die das Beatmungsgerät oder den Defibrillator zu einer Aktion veranlassen. Die Übertragung der Steuersignale erfolgt bevorzugt über das Datenübertragungsmittel.

Mit Hilfe der gewonnen Daten entscheidet das System (Master) situationsabhängig darüber, welche Maßnahmen durch den Notfallhelfer (Slave) durchgeführt werden müssen. Zur Durchführung dieser Maßnahmen wird dann der Notfallhelfer visuell und akustisch durch Sprachausgaben und Textmeldungen oder Piktogramme angeleitet. Die Durchführung dieser Maßnahmen wird durch den Notfallhelfer manuell bestätigt, so dass das System die weiteren Schritte anleiten kann. Die bestätigten manuellen Maßnahmen werden gespeichert und zu den Gesamteinsatzdaten inkl. Zeitstempel hinzugefügt. Zu diesen manuell durch den Anwender durchzuführenden Maßnahmen gehören:
- Durchführung der Herzdruckmassage zum richtigen Zeitpunkt, mit optimaler Frequenz und Drucktiefe an optimaler Position und ausreichender Entlastung
- Analyse des Herzrhythmus und Defibrillation
- intravenöse/intraossären Zugang vorbereiten und legen
- Intubation vorbereiten und durchführen
- Medikamenten-Vorbereitung und Applikation für Reanimationsmedikamente
- therapeutische Hypothermie
- Einstellung der Beatmungsparameter

Bei laufender EKG-Aufzeichnung und -Analyse generiert der Defibrillator ein Steuersignal, das an das Beatmungsgerät übertragen wird, woraufhin dieses die Generierung von Beatmungshüben nach Beendigung der nächsten, auf das Steuersignal folgenden, Exspiration beendet. Der Defibrillator übernimmt die Funktion des Systemmasters.

Dies ist gegebenenfalls erforderlich, weil die Beatmungshübe die EKG-Analyse stören können. Während der Defibrillator eine Schockabgabe vorbereitet und auch schon während des Aufladens, sowie während der Schockabgabe selbst sind die Beatmungshübe weiter unterdrückt. Der Notfallhelfer löst meist den Schock durch Tastendruck nach Aufforderung durch den Systemmaster (Defibrillator) aus. Der Notfallhelfer dient hier als Aktor, der allerdings noch Entscheidungsmöglichkeiten behält.

Die Sensoren des Beatmungsgerätes registrieren einen Atemantrieb des Patienten, woraufhin das Beatmungsgerät eine Rückmeldung an den Defibrillator generiert "Patient atmet". Zusätzlich oder alternativ generiert das Beatmungsgerät über das Display oder andere Signaleinrichtungen eine Information "Patient atmet".

Durch die komplexe Systemkommunikation ist die Datenerfassung und -aufbereitung aller Sensor- und Aktorsignale möglich.

Nachfolgend sind beispielhaft diverse Sensor- beziehungsweise Aktorsignale aufgelistet.
a) Gerätedaten:
   Wartungstermine
   Standort (GPS)
   Betriebsstunden
   Akkustatus
   Funktionskontrollen
   zeitlicher Verlauf (Einschalten, Ausschalten)
b) Monitoring-Modul-Daten:
   EKG (3-/6-/12-Kanal)
   CPR-Feedback-Daten
   HF
   Intrakranielle Druckmessung
   NIBP
   SpO2
   FiO2
   etCO2
   Temperatur
   Alarme, Alarmgrenzen, Ereignisse
   Thoraxkompressionen
   Volumen
   Druck (PEEP, Plateaudruck, Spitzendruck)
c) Beatmungs-Modul-Daten:
   Beatmungsmodus
   Beatmungswerte (AZV, Pinsp. AF, MV, Fi02, Peak, PEEP, CO2)
   Flow O2-Inhalation
d) Defibrillations-Modul-Daten:
   Defibrillation
   Kardioversion
   Automatische EKG-Analyse
   Herz-Schrittmacher

Durchgeführte manuelle Maßnahmen (z.B. Medikamentenapplikation) erfolgen bevorzugt mit Freitextmöglichkeit.

Es können beispielsweise folgende Daten hinzugefügt werden:
Patientendaten (Krankenversicherungskarte, Gesundheitskarte) Einsatz-Daten (Personal, Betreiber, Fahrzeug, Einsatznummer, Einsatzzeiten)
CPR-Thumper-Daten

Zur Integration von Daten anderer Geräte können Protokolle definiert und dokumentiert sein.

Daten können an der Einsatzstelle in einem Protokoll ausgedruckt werden können, um sie dem Weiterbehandelndem zur Verfügung stellen zu können.

Daten können von der Einsatzstelle in Echtzeit zur telemedizinischen Beratung an Supervisor übertragen werden.

Daten können von der Einsatzstelle zur Qualitätssicherung an zentralen Server übertragen werden.

Daten können in gängige Klinikdokumentations-Systeme eingespeist werden können:
Die Alarme und Ansagen beider Geräte werden zu dem Zeitpunkt ausgegeben, an dem sie auftreten (keine Priorisierung der Geräte gegeneinander). Sinnlose Alarme werden damit nicht ausgelöst (die Patientenalarme "Diskonnektion" und "Stenose", die während der Herzdruckmassage in den HLW-Phasen bestehen bleiben, sind während der Analyse-Phase bzw. zur Schockvorbereitung nicht aktiv).

Wird ein vorbereiteter Schock nicht ausgelöst, wird die Beatmung nach erfolgter interner Entladung wieder gestartet.

Die Taste "Maske/Tubus" am Beatmungsgerät behält die Funktion, den Beatmungsdruck auf 20 bzw. 45hPa zu begrenzen. Die Auswahl zwischen "HLW im Rhythmus 30:2" und "Durchdrücken" in den Modi "AED" und "manuell" erfolgt am Defibrillator. Stenose-Alarm wird bei einem Zusammentreffen von Beatmungshub und Herzdruckmassage beim Durchdrücken ausgelöst.

Alarmausgaben können durch intelligente Prozesse auf ein Minimum reduziert werden. Dies bedeutet, dass zur Alarmierung nicht nur die Alarmbedingung, sondern auch einwirkende weitere Parameter/Prozesse überprüft werden.

Die Alarmbedingungen sind bei einer Reanimation zu vernachlässigen, da bekannt ist, dass sich der Patient im reanimationspflichtigen Zustand befindet und dies die einzige Maßnahme ist, um den Bedingungen für die Alarmierung entgegen zu wirken.

Keine Asystolie- oder VF/VT-Alarm, wenn Pulskurve vorhanden.

Visuelle Alarmausgaben sollten einfach und schnell dem alarmierenden Modul zugeordnet werden können.

Akustische Alarmausgaben müssen modulspezifisch sein, um sie dem alarmierenden Modul zuordnen zu können.

Die Lautstärke der Alarmausgabe sollte einstellbar sein und sich automatisch dem Umgebungsgeräuschpegel anpassen.

Die einstellbaren Alarmlimits sollten mit minimalem Aufwand an die patientenspezifischen Bedingungen angepasst werden können (z.B. durch Auto-Alarmlimits).

Die Befehle vom Defibrillator (Master) werden zum Beatmungsgerät (Slave) gesendet.

Der Master erhält bei bestehender Verbindung in jedem Fall eine Rückmeldung zu seinem Befehl.

Folgende Parameter werden bei einer Kommandoübergabe berücksichtigt:
Die Schnittstelle ist auf eine nicht synchronisierte Kommunikation für den Prüfrechner ausgelegt.

Die Firmware des Beatmungsgeräts kann derart erweitert werden, dass Sprache abgeschaltet, Alarme unterdrückt oder beendet, Beatmung gestoppt, gestartet, Stenosealarmgrenze geändert werden können.

Als Parameter kann das Beatmungsgerät zurückliefern, welche Einstellungen (Frequenz), Atemphase oder welcher Alarm anliegt. Für die Darstellung als Monitoringdaten kann der Atemwegsdruck übergeben werden.

Bei der Kommunikation zwischen Defibrillator und Beatmungsgerät übernimmt der Defibrillator die Rolle des Masters. Das Beatmungsgerät arbeitet als Slave und reagiert lediglich auf Befehle, die es vom Defibrillator erhält.

Die Kommunikation wird vom Defibrillator begonnen, indem dieser den entsprechenden Befehl an das Beatmungsgerät sendet. Das Beatmungsgerät schaltet daraufhin seine Sprachausgabe nach einem Hinweis an den Benutzer ab und sendet die entsprechende Antwort an den Defibrillator.

Wenn die Kommunikation unterbrochen wird, während die Beatmung gestoppt ist, setzt das Beatmungsgerät die Beatmung fort, da es keinen neuen Befehl erhält.

Durch den Befehl "start" kann der Defibrillator jederzeit die Unterbrechung der Beatmung aufheben. Das Beatmungsgerät beginnt in diesem Fall sofort, oder nach Beendigung der eventuell anliegenden Insp.-/Exsp.-Phase, mit der Beatmung.

Der Defibrillator überwacht die Anzahl der Beatmungshübe und ob das Beatmungsgerät noch einsatzbereit ist (Demandmodus durch Anwender gewählt).

Die Kommunikation kann vom Defibrillator durch das Senden des Befehls beendet werden. In diesem Fall beendet das Beatmungsgerät das Senden jeglicher Daten, setzt die Beatmung autark fort und bestätigt das Beenden der Kommunikation durch das Befehlsecho.

Sollte das Beatmungsgerät aufgrund eines Übertragungsfehlers den Befehl des Defibrillators nicht verstehen, sendet es einen Befehl zum erneuten Senden. Der Defibrillator wiederholt daraufhin den Befehl. Erfolgt auch nach mehreren Wiederholungen keine erfolgreiche Bestätigung des Befehls durch das Beatmungsgerät, bricht der Defibrillator die Kommunikation ab und beide Geräte arbeiten autark weiter.

Nachdem die Kommunikation durch einen Kommunikationsfehler abgebrochen wurde, fordert der Defibrillator weiterhin die Daten an, solange er keine Antwort vom Beatmungsgerät erhält. Empfängt der Defibrillator eine fehlerhafte Antwort vom Beatmungsgerät, wiederholt er den Befehl bis zu zweimal und anschließend wieder nach 3 s. Durch den Empfang des erwarteten Datenpaketes, wird die Kommunikation wieder hergestellt.

Im Folgenden soll ein komplexes Versorgungssystem am Beispiel einer Unfallsituation beschrieben werden. Das Notfallteam wird zu einem Verkehrsunfall mit einem Fußgänger gerufen. Am Einsatzort angekommen wird versucht den Verletzten anzusprechen und den Unfallhergang zu rekonstruieren. Dabei beschreibt einer der drei Notfallhelfer dem System verbal den Zustand des Patienten und die Situation. Das System speichert diese Daten.

Ein übergeordneter Vor-Ort Supervisor der Einsatzkräfte, welche eine Kontroll- und Überwachungs-Master Einheit zur Überwachung bei sich trägt, bekommt die Daten der Anamnese auf der Master-Einheit angezeigt. Die Notfallhelfer schalten ihre Datenbrillen ein. Die Daten auf der Master-Einheit werden auf die Datenbrillen der einzelnen Einsatzkräfte gesendet und dort angezeigt. Die Master-Einheit des Supervisors fungiert während des Einsatzes als Master und bindet die Datenbrillen und Versorgungssysteme, wie beispielsweise Defibrillator und Beatmungsgerät als Slave mit ein.

Die Datenbrille identifiziert mittels eines Iris-Scan die Notfallhelfer und kennt ab sofort deren Kompetenzen. Die Patientendaten werden fortan auf den Datenbrillen der Einsatzkräfte angezeigt. Die Notfallhelfer beginnen mit dem Monitoring des Patienten. Die Notfallhelfer schließen Sensoren zum Messen der Vitalparameter, wie Sauerstoffsättigung, Blutdruck und Puls an den Patienten an. Darüber hinaus wird ein EKG an den Patienten angeschlossen.

Die Daten werden über die Master-Einheit an den Vor-Ort Supervisor zu den einzelnen Datenbrillen der Notfallhelfer gesendet und angezeigt. Da der Zustand des Patienten sich verschlechtert, gibt das System individuell an den Supervisor und an die Notfallhelfer Warnungen ab.

Die Notfallhelfer bemerken, dass der Patient eintrübt, die Atmung zunehmend schwächer wird und deutliche Rasselgeräusche beim Atmen hörbar sind. Die aufgenommenen Vitalparameter werden laufend an die Master-Einheit des Supervisors gesendet. Dieser nimmt, aufgrund der Situation des Patienten, Kontakt mit einem Experten in der nächsten Notfallklinik auf. Die Daten des Patienten werden dabei in Echtzeit an den Experten in der Notfallklinik gesendet.

Aufgrund des schlechter werdenden Blutdruckes des Patienten, werden weitere Maßnahmen von dem Experten empfohlen. Wegen des Verdachtes eines Schädel-Hirn-Traumas im Rahmen des Verkehrsunfalls, wird eine intrakranielle Druckmessung am Schädel des Patienten durchgeführt. Ebenso wird der Abdominalraum des Patienten mithilfe einer Ultraschall-Untersuchung durchgeführt. Die Bilder der beiden Systeme werden mittels kabelloser Datenübertragung auf die Datenbrillen der Notfallhelfer und des Supervisors übertragen.

Es werden Einblutungen festgestellt. Der Zustand des Patienten verschlechtert sich weiterhin, bis er reanimationspflichtig wird. Die Notfallhelfer beginnen sofort mit der Reanimation und entscheiden sich zur Intubation. Mithilfe eines Videolaryngoskop wird ein Videobild des Rachens des Patienten auf die Datenbrille eingespielt. Aufgrund der schwer zugänglichen Lage kann der Patient mit Hilfe des Videobildes intubiert werden. Über Sprachbefehle wird die Absaugpumpe aktiviert. Sobald der Tubus sitzt wird das Beatmungsgerät per Sprachbefehle aktiviert.

Der Atemzustand wird überwacht und die Daten werden auf die Master-Einheit und auf die Datenbrillen der Notfallhelfer gespielt. Alle Maßnahmen werden durch eine Kamera in den Datenbrillen der Notfallhelfer erkannt und im Dokumentenspeicher der Master Einheit gespeichert. Diese werden an einen zentralen Sicherheits-Server übermittelt zur späteren Abrechnung des Einsatzes und zur Qualitätssicherung und -verbesserung der eingesetzten Kräfte.

Das System erkennt während der Reanimation, welche Maßnahmen bereits durch die Notfallhelfer durchgeführt worden sind und gibt mithilfe Algorithmus basierter Auswertung, darauf situationsabhängig Empfehlungen für die weiteren therapeutischen Möglichkeiten. Die Helfer entscheiden sich, den Patienten schnellstmöglich in die nächste Notfall- und Traumaklinik zu transportieren. Da der Patient weiterhin reanimationspflichtig ist, entscheiden sich die Einsatzkräfte ein automatisches Reanimationsgerät für die Fahrt zum Krankenhaus einzusetzen.

Die Steuerung des Reanimationsgerätes wird kabellos von dem Versorgungssystem übernommen, sodass eine optimale Abfolge zwischen Beatmung und Herzdruckmassagen ablaufen kann. In diesem Moment übernimmt der Defibrillator des Versorgungssystems die Rolle des Masters, um Beatmungsgerät und Reanimationsgerät zu steuern. Das Versorgungssystem erkennt den Zustand des Patienten und gibt während des Einsatzes Sprachausgaben mit weiteren Handlungsempfehlungen. Da der Vor-Ort Supervisor bereits mittels telemedizinischer Unterstützung die Daten an den Experten im Krankenhaus gesendet hat, kann das Krankenhaus sich auf die Ankunft des Patienten optimal vorbereiten.

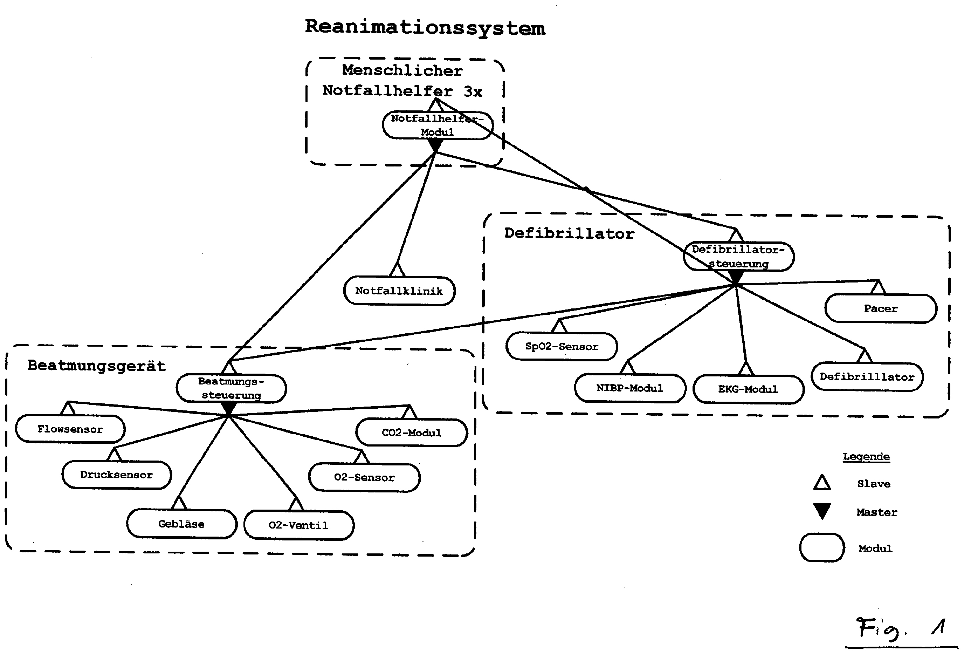

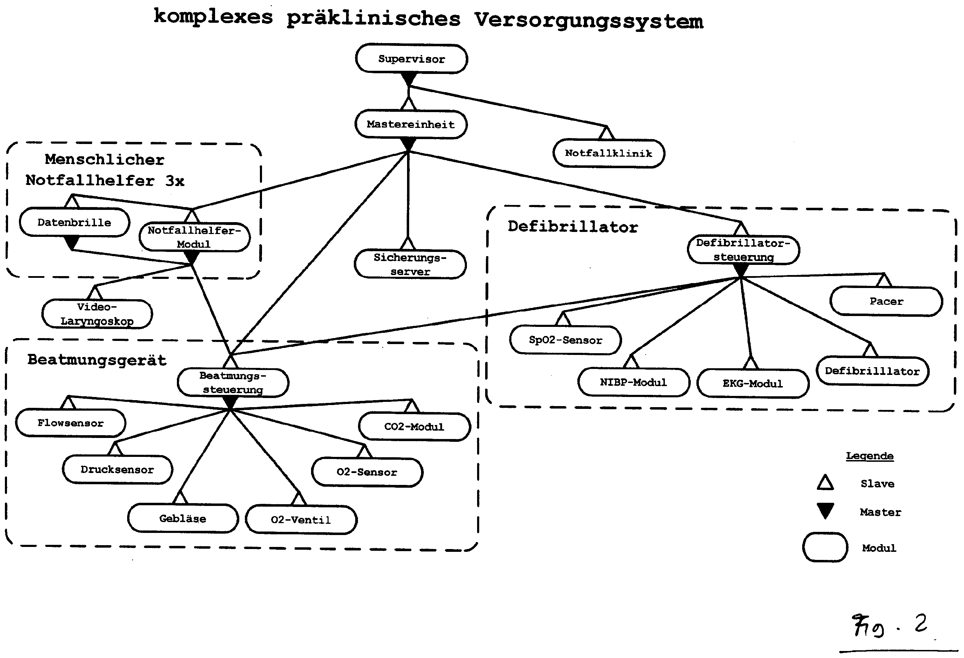

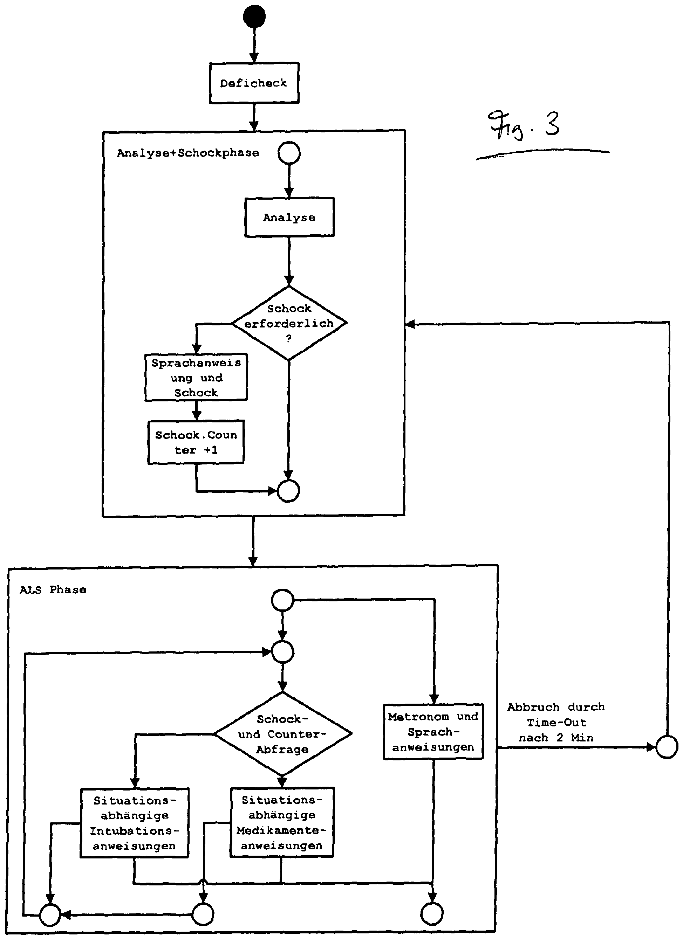

## Patentansprüche

1. Vorrichtung zur präklinischen Notfallversorgung, in der aus mindestens zwei miteinander vernetzten Modulen ein Netzwerk ausgebildet ist und wobei die Module durch die Vernetzung einen situationsabhängigen Handlungsablauf ermöglichen, **dadurch gekennzeichnet, dass** zur Ausbildung eines Systems ein Modul ein Defibrillator und eines ein Beatmungsgerät ist und dass das System zustandsabhängig steuert oder regelt, wobei die Module für die Ablaufsteuerung eine Organisationsstruktur aufweisen und wobei die Organisationsstruktur Master-Slave-Prinzipien derart beinhaltet, dass der/die Systemmaster während der Versorgung zwischen dem Defibrillator und dem Beatmungsgerät wechseln und wobei die Systemmaster über die Alarmausgabe wachen und sie situationsabhängig steuern.

2. Vorrichtung nach Anspruch 1, **dadurch gekennzeichnet, dass** die Module Sensoren, Aktoren, Eingabe-, Ausgabeelemente und/ oder Steuer-/ Regelungselemente enthalten.

3. Vorrichtung nach Anspruch 1, **dadurch gekennzeichnet, dass** das Netzwerk drahtgebunden, drahtlose, optische, akustische, magnetische und/ oder taktile Übertragungstechniken nutzt.

4. Vorrichtung nach Anspruch 1, **dadurch gekennzeichnet, dass** das Netzwerk eindeutige Modul/ Daten-Identifikation mit Zeitstempel und abgesicherte Kommunikation ermöglicht.

5. Vorrichtung nach Anspruch 1, **dadurch gekennzeichnet, dass** bei Kommunikationsabbruch unter- und übergeordneten Module autark weiterarbeiten.

6. Vorrichtung nach Anspruch 1, **dadurch gekennzeichnet, dass** eine intelligente, situationsabhängige Sprachführung einen menschlichen Helfer unterstützt.

7. Vorrichtung nach Anspruch 1, **dadurch gekennzeichnet, dass** sich Videolaryngoskope, CPR-Feedbacksysteme, Fernbedienungsmodule, Ultraschallsysteme, Hirndruckmessungssysteme, Datenbrillen, Dokumentationssysteme und/oder anderweitige Sensor-, Aktor und/ oder HMI-Module in die Vorrichtung mit einbinden lassen.

8. Vorrichtung nach Anspruch 1, **dadurch gekennzeichnet, dass** Master-Slave-Unterstrukturen als Slave für übergeordnete Organisationsstrukturen dienen.

9. Vorrichtung nach Anspruch 1, **dadurch gekennzeichnet, dass** es mehrere parallele, gleichberechtigte Organisationsstrukturen gibt.

10. Vorrichtung nach Anspruch 1, **dadurch gekennzeichnet, dass** Module während der Maßnahme dem System zugeführt oder aus dem System entfernt werden können.

11. Vorrichtung nach einem der Ansprüche 1 bis 10, **dadurch gekennzeichnet, dass** das System den Therapievorgang benutzergesteuert und/ oder selbsttätig optimiert.

## Claims

1. Device for preclinical emergency care, in which a network is formed from at least two interlinked modules, and wherein the modules permit a situation-dependent course of action through the interlinkage, **characterized in that**, in order to form a system, one module is a defibrillator and one a ventilator, and **in that** the system controls or regulates in a condition-dependent manner, wherein the modules have an organizational structure for the sequence control, and wherein the organizational structure contains master-slave principles, in such a way that the system master(s) alternate(s) between the defibrillator and the ventilator during the care, and wherein the system masters monitor the alarm output and control the latter in a situation-dependent manner.

2. Device according to Claim 1, **characterized in that** the modules contain sensors, actuators, input elements, output elements and/or open-loop/closed-loop control elements.

3. Device according to Claim 1, **characterized in that** the network uses wired, wireless, optical, acoustic, magnetic and/or tactile transmission techniques.

4. Device according to Claim 1, **characterized in that** the network permits unique module/data identification with time stamp and secured communication.

5. Device according to Claim 1, **characterized in that**, if communication is interrupted, lower-level and higher-level modules continue to work autonomously.

6. Device according to Claim 1, **characterized in that** an intelligent, situation-dependent voice prompt assists a human helper.

7. Device according to Claim 1, **characterized in that** video laryngoscopes, CPR feedback systems, remote-control modules, ultrasound systems, intracranial pressure measurement systems, smart glasses, documentation systems and/or other sensor, actuator and/or HMI modules can be integrated into the device.

8. Device according to Claim 1, **characterized in that** master-slave lower-level structures serve as slaves for higher-level organizational structures.

9. Device according to Claim 1, **characterized in that** there are a plurality of parallel, equally authorized organizational structures.

10. Device according to Claim 1, **characterized in that** modules can be added to the system or removed from the system during the measure.

11. Device according to one of Claims 1 to 10, **characterized in that** the system optimizes the therapy process in a user-controlled and/or autonomous manner.

## Revendications

1. Dispositif d'alimentation de secours préclinique, dans lequel un réseau est formé à partir d'au moins deux modules interconnectés en réseau et les modules, grâce à la connexion en réseau, rendant possible une procédure dépendante de la situation, **caractérisé en ce que** pour former un système, un module est un défibrillateur et un autre un appareil respiratoire et **en ce que** le système commande ou régule en fonction de l'état, les modules présentant une structure d'organisation pour la commande du déroulement et la structure d'organisation contenant des principes maître-esclave de telle sorte que le/les maîtres du système alternent pendant l'alimentation entre le défibrillateur et l'appareil respiratoire et les maîtres du système veillent par le biais de la sortie d'alarme et les commandent en fonction de la situation.

2. Dispositif selon la revendication 1, **caractérisé en ce que** les modules contiennent des capteurs, des actionneurs, des éléments d'entrée, de sortie et/ou des éléments de commande/régulation.

3. Dispositif selon la revendication 1, **caractérisé en ce que** le réseau utilise des techniques de transmission filaires, sans fil, optiques, acoustiques, magnétiques et/ou tactiles.

4. Dispositif selon la revendication 1, **caractérisé en ce que** le réseau rend possible une identification univoque de module/données avec une estampille temporelle et une communication sécurisée.

5. Dispositif selon la revendication 1, **caractérisé en ce qu'**en cas de rupture de communication, les modules subordonnés et superviseurs continuent de fonctionner de manière autonome.

6. Dispositif selon la revendication 1, **caractérisé en ce qu'**un guidage vocal indépendant dépendant de la situation assiste un auxiliaire humain.

7. Dispositif selon la revendication 1, **caractérisé en ce que** des vidéolaryngoscopes, des systèmes de rétroaction de réanimation cardiopulmonaire, des modules de commande à distance, des systèmes à ultrasons, des systèmes de mesure de la pression cérébrale, des lunettes connectées, des systèmes de documentation et/ou d'autres types de modules capteurs, actionneurs et/ou HMI peuvent être intégrés dans le dispositif.

8. Dispositif selon la revendication 1, **caractérisé en ce que** des sous-structures maître-esclave servent d'esclaves pour des structures d'organisation de niveau supérieur.

9. Dispositif selon la revendication 1, **caractérisé en ce qu'**il existe plusieurs structures d'organisation parallèles égales.

10. Dispositif selon la revendication 1, **caractérisé en ce que** des modules peuvent être acheminés au système ou retirés du système pendant la mesure.

11. Dispositif selon l'une des revendications 1 à 10, **caractérisé en ce que** le système optimise l'opération thérapeutique en étant commandé par l'utilisateur et/ou automatiquement.
